# EUROPEAN PATENT APPLICATION

(11) **EP 4 193 920 A1**
(43) Date of publication of application: **14.06.2023**
(21) Application number: 21213304.5
(22) Date of filing: 09.12.2021
(51) Int. Cl.: A61B 5/08, A61B 5/087, A61B 5/00, A61B 5/113, A61B 5/11, A61B 7/00, A61B 5/024, A61B 5/1455, A61B 5/091, A61M 16/00

(54) **A SYSTEM AND METHOD FOR MONITORING COPD PATIENTS**

(71) Applicant: Koninklijke Philips N.V., 5656 AG Eindhoven (NL)
(72) Inventor: CHAKRABARTI, Biswaroop, Eindhoven (NL); KATIPALLY, Karthik Raj, Eindhoven (NL); BERA, Deep, Eindhoven (NL); HIWALE, Sujitkumar, Eindhoven (NL); RADHAKRISHNAN, Ravindranath, Eindhoven (NL)
(74) Representative: Philips Intellectual Property & Standards

(57) **Abstract**

A system is for monitoring COPD patients having (at least) a microphone for monitoring the patient's breathing sounds, an accelerometer for monitoring the patient's movements and a pulse oximetry sensor. A state of arousal or of sleep is estimated and sleep stages are estimated based on the microphone and accelerometer signals. Signals are also received from a positive applied pressure, PAP, system indicating respiratory flow information and 02 and/or CO2 concentrations. The PAP system signals are allocated to the estimated sleep stage and an indicator of COPD exacerbation is derived based on the relationship between the sleep stage the monitored signals.

## Description

### FIELD OF THE INVENTION

The invention relates to a patient monitoring system, in particular for monitoring patients with chronic obstructive pulmonary disease.

### BACKGROUND OF THE INVENTION

Chronic obstructive pulmonary disease (COPD) is a progressive life threatening disorder characterized by chronic airflow obstruction manifesting itself as breathlessness (initially with exertion) and often leads to exacerbations and serious illness. It is estimated that more than 250 million people are suffering from COPD, and it is responsible for more than 3 million deaths (5% of all reported deaths globally in 2015).

COPD prevalence is likely to increase in the coming years due to a high smoking prevalence and aging populations (due to increased life expectancy). COPD is usually diagnosed based on medical history and physical examination and is confirmed by a pulmonary function test.

The natural history of COPD is characterized by periods of low symptoms with episodes of worsening. An acute exacerbation of chronic obstructive pulmonary disease or acute exacerbations of chronic bronchitis (AECB), is a sudden worsening of COPD symptoms including shortness of breath, quantity and color of phlegm that typically lasts for several days. Usually an exacerbation is triggered by environmental pollutants, allergens or an infection. Exacerbations are classified as mild, moderate and severe and they become more frequent for a patient as the disease progresses. In addition to causing discomfort to the patient, such as increased breathlessness (possibly requiring therapy), severe productive cough, fever & chills etc., such exacerbation may cause other conditions, such as spontaneous [traumatic] pneumothorax.

The short term prediction of COPD exacerbation is difficult, for example in the time frame of days to weeks.

### SUMMARY OF THE INVENTION

The invention is defined by the claims.

According to examples in accordance with an aspect of the invention, there is provided a system for monitoring COPD patients, comprising:
a microphone for monitoring the patient's breathing sounds;
an accelerometer for monitoring the patient's movements;
a pulse oximetry sensor;
a processor which is adapted to:
   estimate a state of arousal or of sleep and estimate sleep stages based on the microphone and accelerometer signals;
   receive signals from a positive applied pressure, PAP, system indicating:
      respiratory flow information; and
      O2 and/or CO2 concentrations;
      allocate the PAP system signals to the estimated sleep stage; and
      provide an indicator of COPD exacerbation based on the relationship between (i) the estimated sleep stage and (ii) the PAP system signals and pulse oximetry sensor signals.

In this system, staging of the COPD patient's sleep is performed using microphone and accelerometer data, which may for example be obtained from a mobile phone. The additional PAP system signals and pulse oximetry signals, relating to the respiratory system and other physiological parameters, can then be annotated as associated with specific sleep stages.

For different patients with different COPD conditions, there is a different relationship between their physiological parameters (including those measured by a PAP system) when in different stages of sleep. It is noted that "different COPD conditions" may refer to classification of the disease into chronic bronchitis or emphysema for example, or it may relate to the severity of symptoms. Thus, by analyzing the sleep stages of a patient and the corresponding (i.e. at the same time) physiological signals, it becomes possible to predict their COPD condition and hence provide an early indication of COPD exacerbation.

The system may additionally make use of self-reported signs and symptoms when awake. The sleep analysis may also detect and count apnea events.

The system may further comprise a memory storing a set of sleep stage predictor models, each model predicting a sleep stage from the PAP system signals and pulse oximetry sensor signal, wherein the different models of the set relate to patients with different COPD conditions. Thus, by matching a patient to the closest model, it is then determined which COPD conditions most closely match the patient.

Sleep stage predictors may be constructed from the physiological parameters for various population groups, with different COPD conditions. Thus, the way the physiological parameters map to different sleep stages for patients with different COPD conditions is used to determine COPD conditions of a patient based on their measured parameters and sleep stages.

The sleep stage predictor models may for example be constructed from a well-defined population (e.g. one predictor model may be for asymptomatic COPD patients within a given age range, of a specific gender and within a BMI range with no comorbidities). Different predictor models will be for different categories of patient. The predictor model will perform well for a subject matching the group's specification (e.g. a post-exacerbation COPD patient) but will perform worse for another subject (e.g. a pre-exacerbation COPD patient). Conversely, a separate model constructed from a group of pre-exacerbation COPD patients should perform better for the latter and worse for the former subject.

Thus, by matching a patient to the best sleep stage predictor, an estimate of COPD exacerbation probability can be derived from these predictors. In the case of an impending exacerbation, the current best model for the patient will start to de-correlate with the sleep staging and the correlation of a different model (e.g. one for an exacerbation-specific model) will improve.

The microphone and accelerometer are for example part of a smart phone.

The PAP system signals may further indicate tidal volume. They may instead or additionally indicate FeO2 and/or FeCO2 and/or Fi02 and/or FiCO2,

The pulse oximetry sensor for example also measures heart rate.

The respiratory flow information for example comprises one or more of:
tidal volume;
respiration rate;
respiratory flow rate.

The processor may be adapted to derive heart rate variability data from heart rate data and optionally derive a respiration rate from the sinus arrhythmia identification from the heart rate variation.

Thus, by monitoring additional parameters, the prediction of a COPD condition becomes increasingly reliable.

The system may further comprise one or more of:
an EEG headband;
an ECG system; and
an EOG system,
wherein the processor is further adapted to estimate sleep stages using the EEG, ECG and/or EOG signals.

This provides improved sleep stage estimation.

The system may further comprise a camera to monitor chest and/or abdomen movement and the processor is adapted to provide an estimation of tidal volume through remote PPG sensing and to monitor limb and trunk movements. These may again make the sleep stage estimation more robust.

The system may further comprise an EMG monitoring system for monitoring limb movement

The invention also provides a PAP system, comprising:
a patient mask;
a pressure source for delivering breathing gas to the patient mask; and
the monitoring system as defined above.

The invention also provides a monitoring method for monitoring COPD patients, comprising:
receiving a signal indicating the patient's breathing sounds;
receiving a signal indicating the patient's movements;
receiving a pulse oximetry signal;
estimating a state of arousal or of sleep and estimating sleep stages based on the microphone and accelerometer signals;
receive signals from a positive applied pressure, PAP, system indicating:
   respiratory flow information; and
   O2 and/or CO2 concentrations;
   allocate the PAP system signals to the estimated sleep stage; and
   provide an indicator of COPD exacerbation based on the relationship between the estimated sleep stage and the PAP system signals.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for monitoring COPD patients; and
Figure 2 shows a method for monitoring COPD patients.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for monitoring COPD patients having (at least) a microphone for monitoring the patient's breathing sounds, an accelerometer for monitoring the patient's movements and a pulse oximetry sensor. A state of arousal or of sleep is estimated and sleep stages are estimated based on the microphone and accelerometer signals. Signals are also received from a positive applied pressure, PAP, system indicating respiratory flow information and O2 and/or CO2 concentrations. The PAP system signals are allocated to the estimated sleep stage and an indicator of COPD exacerbation is derived based on the relationship between the sleep stage the monitored signals.

The invention is based on the appreciation that a study of sleep disturbances in COPD patients may be of interest for predicting COPD exacerbation, as there are numerous reports of nocturnal and sleep related symptoms being associated with COPD.

Sleep disorders are typically studied through polysomnography which is a multi-parametric test combining EEG, ECG, EMG, EOG, respiratory functions and pulse oximetry etc. However, polysomnography is a multi-parametric test which requires a sleep laboratory and typically not feasible to set up at the patient's home. Many patients are tested using a home sleep test, which is a pared down version of polysomnography and captures fewer data channels such as oxygen saturation, heart rate, airflow, chest and abdomen movements, snoring and sleep position.

However, these tests are mainly geared towards sleep apnea and as such not directly suitable for COPD exacerbation prediction. They may necessitate use of devices that are otherwise not required by a COPD patient involving additional cost and discomfort. They also provide insufficient information compared to polysomnography.

The inventors have found that analysis of sleep related parameters, even with a set of sensors suitable for a home test, can yield useful information for predicting exacerbation. In addition, there is an identified overlap syndrome where COPD patients suffer from an additional co-morbidity of sleep apnea, and the invention can be applied in such cases as well. Monitoring of sleep related symptoms and the ability to identify an exacerbation is of great benefit in such patients.

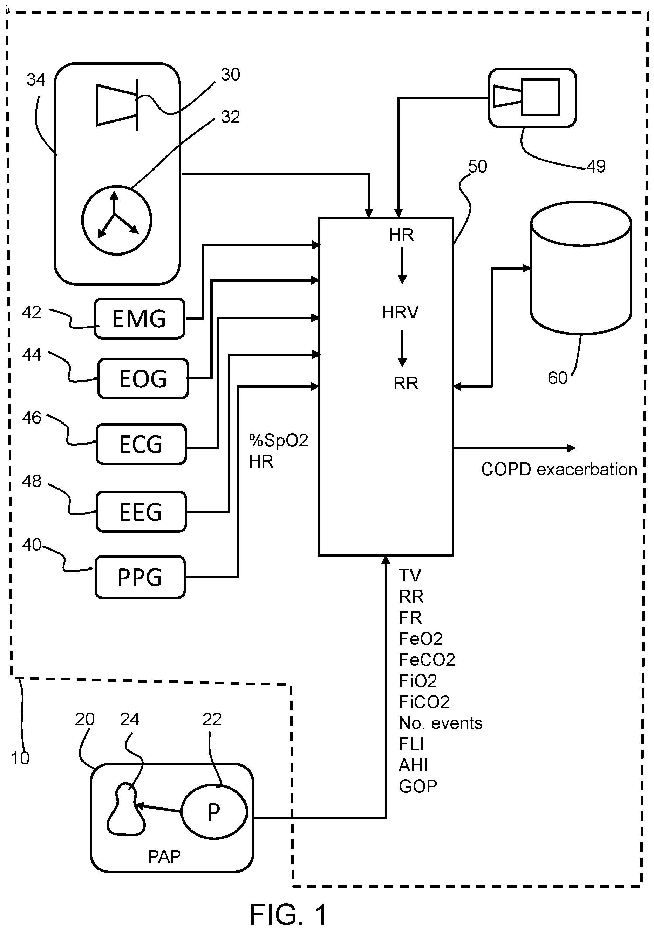

Figure 1 shows a system 10 for monitoring COPD patients to be used in conjunction with a positive airway pressure (PAP) system 20. The PAP system 20 comprises a pressure source 22 and a mask 24. It may comprise a continuous PAP (CPAP) system, a bilevel PAP (BiPAP) system or an automatic PAP (APAP) system.

The system is shown with a full set of possible sensing inputs.

The most basic set however comprises:
a microphone 30 for monitoring the patient's breathing sounds;
an accelerometer 32 for monitoring the patient's movements;
a pulse oximetry sensor 40 (which measures SpO2 and HR); and
signals from the PAP system 20.

The system has a processor 50 which process the various sensor inputs. The processor 50 estimates a state of arousal or of sleep and estimates sleep stages based on at least the microphone 30 and accelerometer 32 signals (and any additional sensing modalities, if provided).

The most basic set of signals from the positive applied pressure, PAP, system indicate:
respiratory flow information (e.g. one or more of tidal volume TV, respiration rate RR, respiration flow rate FR; and
O2 and/or CO2 concentrations (e.g. one or more of expired O2 fraction FeO2, expired CO2 fraction FeCO2, inspired O2 fraction Fi02 and inspired CO2 fraction FiCO2).

The processor allocates the PAP system signals to the estimated sleep stage.

A relationship between the estimated sleep stage (obtained from the microphone and accelerometer) and the other sensor signals (PAP signals and pulse oximetry sensor signals) can be used to determine a level of COPD exacerbation.

The sleep staging of the COPD patient is performed using the microphone and accelerometer data, which may for example be obtained from a mobile phone 34. The additional PAP system signals and pulse oximetry signals, relating to the respiratory system and other physiological parameters, can then be annotated as associated with specific sleep stages.

For different patients with different COPD conditions, there is a different relationship between their physiological parameters (including those measured by a PAP system) when in different stages of sleep. Thus, by analyzing the sleep stages of a patient and the corresponding (i.e. at the same time) physiological signals, it becomes possible to predict their COPD condition and hence provide an early indication of COPD exacerbation.

The PAP system may additionally provide a flow limitation index FLI, a number of apnea events ("No. events"), an apnea-hypopnea index HPI and a glottis opening pressure GOP. Some or all of these may be used to improve the sleep stage detection.

Other optional sensors are shown in Figure 1, including an EMG system 42 for monitoring limb movement based on limb muscle EMG signals, an EOG system 44, an ECG system 46, an EEG system 48 (such as an EEG headband to monitor brainwaves for accurate sleep staging) and a camera system 49. The camera system is for example a thermal camera able to monitor chest and abdomen movement and provide its own estimation of tidal volume through remote photoplethysmography (PPG).

The system may also receive and process self-reported signs and symptoms.

The processor accesses a memory 60 which stores a set of sleep stage predictor models, each model predicting a sleep stage from the PAP system signals and pulse oximetry sensor signal (and using any of the additional optional sensor signals). The different models of the set relate to patients with different COPD conditions as well as different patient parameters (gender, age, BMI etc.).

In this way, sleep stage predictors are constructed from the physiological parameters for various population groups, with different COPD conditions. Thus, the way the physiological parameters map to different sleep stages for patients with different COPD and personal conditions is used to determine COPD conditions of a patient based on their measured parameters and sleep stages.

The mobile phone is for example placed on the bed close to the patient to monitor the patient's breath sounds (including snoring) and movements to estimate state of arousal or sleep and apnea. Alternatively, a smart watch may be used to collect the this data, as well as the pulse oximetry data (by means of a PPG sensor).

The invention makes use of the sensor data by a three stage process.

Firstly, as part of an initial system configuration, sleep stage predictors are constructed from the physiological parameters for various population groups. A sleep stage predictor algorithm constructed from a well-defined population will be expected to perform well for a subject matching the group's specification but would perform worse for another subject. The population groups are for example defined by their current level of symptoms, their age, gender and BMI, and any comorbidities.

Secondly, the staging of the COPD patient's sleep is performed with the microphone and accelerometer data obtained from the mobile phone, and the respiratory system related parameters and other physiological parameters are annotated as belonging to specific sleep stages.

Finally, a COPD exacerbation probability is estimated by comparing the performance (in particular correlation with actual sleep stage) of these predictors.

Some specific implementation examples will now be presented.

A first example of the system makes use of a mobile phone microphone and accelerometer, with SpO2, Fi02, FeO2 and tidal volume data. The Fi02 and FeO2 (and optionally also FiCO2 and FeCO2) measurements are made by a device attached at the level of the PAP mask.

Accurate sleep staging requires brainwave analysis (EEG). However, the use of the microphone and accelerometer present in a mobile phone placed on the bed close to the patient is able to construct sleep-staging information sufficient for this application. This sleep staging approach is known and has been demonstrated to be feasible.

The respiratory flow and volume parameters as measured by the PAP system and the O2 and CO2 concentration in inhaled and exhaled air are used in addition to the SpO2 as measured by pulse oximetry.

The basic working principle of this version of the system is now explained.

### 1. Population model creation

Between two subjects with different disease affection, the overall pattern of different sleep stages ('sleep architecture') differs; and the parameters sampled in the same sleep stage (e.g. REM sleep) would exhibit differences as well. For example, reference is made to E. Choi, D.-H. Park, J. Yu, S.-H. Ryu, and J.-H. Ha, "The Severity of Sleep Disordered Breathing Induces Different Decrease in the Oxygen Saturation During Rapid Eye Movement and Non-Rapid Eye Movement Sleep," Psychiatry Investig, vol. 13, no. 6, p. 652, 2016. This shows significant SpO2 differences among different OSA grades in both REM and NREM sleep. Reference is also made to M. El-Sayed Ashour, M. El-Hafez Zedan, and A. Farahat, "Sleep pattern changes in chronic obstructive pulmonary disease patients," Egypt J Chest Dis Tuberc, vol. 67, no. 2, p. 99, 2018. This shows differences in REM & NREM sleep percentage, sleep efficiency, and apnea-hypopnea statistics in different COPD grades.

It has been found that such differences are also observed for COPD patients with different symptom levels. For example, the weighing and combination rules of the primary parameters may vary between COPD patients in a low-symptom period and those in the pre-exacerbation period. The model generation may make involve a cohort of COPD patients, so pre-exacerbation period data can be isolated by defining a suitable time interval before an exacerbation episode as pre-exacerbation period and analyzing data from those periods. Accounting for demographic variations, this would be the most important sub-population definition.

Other classifications may involve:
(i) A COPD-free group and COPD-affected group;
(ii) Age stratified population groups; and
(iii) Population groups stratified by gender and other demographic parameters.

A small number of alternate population models may for example be created which would be deployed in parallel. Thus, a knowledge dataset is constructed with different population groups.

The sleep stage predictors are constructed for each subpopulation with the measured and derived respiratory and other physiological parameters as features.

A population stratification is observed and the model differs between subpopulations, for example mean SpO2 at NREM sleep stage 2 would typically be different for the two groups of subjects, and similar trends are observed for other parameters. The sleep pattern will differ as well.

If an individual model is based on a linear combination of the features, the weighing coefficient values would change between the subpopulations.

### 2. Data collection

The subject (a COPD patient using a CPAP/BiPAP/APAP device) would proceed to sleep wearing the mask of the PAP device. A gas sensing device is attached to the mask beforehand and continually estimates Fi02 and FeO2 and transmits the timestamped recordings to the processor and its associated software.

Timestamped tidal volume and flow recordings from the PAP device are also sent to the processor and associated software. The SpO2 and heart rate data are similarly recorded.

At a first level of processing in software, a set of secondary parameters can be estimated from these primary sensed parameters. Some example derived secondary parameters are:
Heart rate variability HRV data from the heart rate data;
Respiratory rate RR from the sinus arrhythmia identification from the heart rate variation HRV;
Time averaged (moving window) and smoothed blood oxygen and carbon dioxide concentrations.

The data from all sources is synchronized.

Some parameters may be estimated with redundancy, such as the respiratory rate which could be directly recorded as well as imputed from heart rate variability data (through sinus arrhythmia detection), Thus, in the case of missing data from one of the sources, a continuous data stream can still be reconstructed.

A basic sleep staging is initially performed from the activity data available through the microphone and accelerometer measurements with known sleep scoring algorithms.

### 3. System deployment

After the population-specific predictors are constructed, the processor is used to provide short-term prediction of COPD exacerbation events. For a COPD patient, usually the features corresponding to exacerbation free period would be expected to correlate best with the sleep staging if there is no probability of exacerbation. However, the following pattern is observed in case of an impending exacerbation:
(i) In a period leading up to an exacerbation, the model (suited to an exacerbation-free period) will start to decorrelate with the sleep staging (i.e. the performance of the model in predicting REM and NREM sleep stages and awake periods would suffer).
(ii) The correlation between the actual sleep staging with that predicted by exacerbation-specific model correlation will however improve. In addition, the pattern of the hypnogram will shift to indicate poorer overall quality of sleep.

This change in the pattern of correlations, along with a deterioration of sleep quality, will thereby raise a suspicion of an impending exacerbation.

As mentioned above, the model may be augmented with self-reported symptoms. It may thus be personalized with trending information. The patient-reported symptoms for example indicate:
Breathlessness;
Cough (dry or productive, and if productive, quality and quantity of sputum);
Sleep quality;
Daytime somnolence;
Tiredness; or
Fever.

The categorical and subjective parameters are numerically recoded so that an objective recording can be obtained. Alternately, an established questionnaire suitable for the problem may be leveraged and the responses appropriately recoded for objective analysis.

As also mentioned above, the accuracy of the sleep staging may be improved with additional electrophysiology data, which may include one or more of:
EEG: multichannel electroencephalogram recording, which may be achieved by scalp electrodes or an EEG headband;
ECG: Preferably a portable patch instead of multi-electrode/multichannel recording;
EMG (limb muscles, diaphragm, and intercostal muscles): In a minimal setup, intercostal muscle EMG can be integrated with an ECG using a chest patch; and
EOG (electrooculogram).

These components would be used solely for the sleep staging and consequent annotation of the respiratory system related and other physiological parameters.

The camera monitoring (visible light or thermal) mentioned above allows the subject's movements to be monitored for further improvement of the sleep staging.

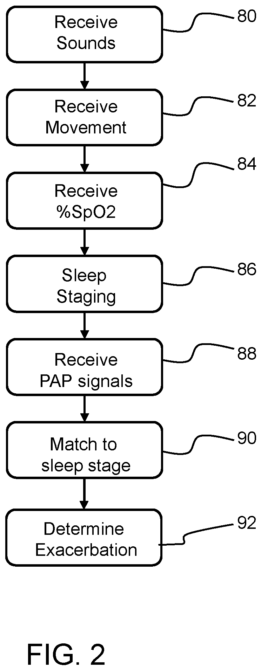

Figure 2 shows a monitoring method for monitoring COPD patients, comprising:
in step 80, receiving a signal indicating the patient's breathing sounds;
in step 82, receiving a signal indicating the patient's movements;
in step 84, receiving a pulse oximetry signal.

In step 86, a state of arousal or of sleep is estimated and sleep stages are estimated based on (at least) the microphone and accelerometer signals.

Signals are also received from a positive applied pressure, PAP, system in step 88. These indicate respiratory flow information and O2 and/or CO2 concentrations.

The PAP system signals are allocated to the estimated sleep stage in step 90 so that an indicator of COPD exacerbation can be provided in step 92 based on the relationship between the estimated sleep stage and the PAP system signals.

The invention is of interest for predicting an impending exacerbation for a COPD patient (and/or worsening of symptoms of an OSA/overlap syndrome patient) in a short-term basis and help decide management to ameliorate or altogether avoid the exacerbation.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (10) for monitoring COPD patients, comprising:
a microphone (30) for monitoring the patient's breathing sounds;
an accelerometer (32) for monitoring the patient's movements;
a pulse oximetry sensor (40);
a processor (50) which is adapted to:
estimate a state of arousal or of sleep and estimate sleep stages based on the microphone and accelerometer signals;
receive signals from a positive applied pressure, PAP, system indicating:
respiratory flow information; and
O2 and/or CO2 concentrations;
allocate the PAP system signals to the estimated sleep stage; and
provide an indicator of COPD exacerbation based on the relationship between (i) the estimated sleep stage and (ii) the PAP system signals and pulse oximetry sensor signals.

2. The system of claim 1, further comprising a memory (60) storing a set of sleep stage predictor models, each model predicting a sleep stage from the PAP system signals and pulse oximetry sensor signal, wherein the different models of the set relate to patients with different COPD conditions.

3. The system of claim 1 or 2, wherein the microphone and accelerometer are part of a smart phone (34).

4. The system of any one of claims 1 to 3, wherein the PAP system signals further indicate tidal volume.

5. The system of any one of claims 1 to 4, wherein the PAP system signals indicate FeO2 and/or FeCO2.

6. The system of any one of claims 1 to 5, wherein the PAP system signals indicate Fi02 and/or FiCO2,

7. The system of any one of claims 1 to 6, wherein the pulse oximetry sensor also measures heart rate.

8. The system of any one of claims 1 to 7, wherein the respiratory flow information comprises one or more of:
tidal volume;
respiration rate;
respiratory flow rate.

9. The system of any one of claims 1 to 8, wherein the processor (60) is adapted to derive heart rate variability data from heart rate data and optionally derive a respiration rate from the sinus arrhythmia identification from the heart rate variation.

10. The system of any one of claims 1 to 9, further comprising one or more of:
an EEG headband (48);
an ECG system (46); and
an EOG system (44),
wherein the processor (60) is further adapted to estimate sleep stages using the EEG, ECG and/or EOG signals.

11. The system of any one of claims 1 to 10, further comprising a camera (49) to monitor chest and/or abdomen movement and the processor is adapted to provide an estimation of tidal volume through remote PPG sensing and to monitor limb and trunk movements.

12. The system of any one of claims 1 to 11, further comprising an EMG monitoring system (42) for monitoring limb movement

13. A PAP system, comprising:
a patient mask (24);
a pressure source (22) for delivering breathing gas to the patient mask; and
the monitoring system (10) of any one of claims 1 to 12.

14. A monitoring method for monitoring COPD patients, comprising:
(80) receiving a signal indicating the patient's breathing sounds;
(82) receiving a signal indicating the patient's movements;
(84) receiving a pulse oximetry signal;
(86) estimating a state of arousal or of sleep and estimating sleep stages based on the microphone and accelerometer signals;
(88) receive signals from a positive applied pressure, PAP, system indicating:
respiratory flow information; and
O2 and/or CO2 concentrations;
(90) allocate the PAP system signals to the estimated sleep stage; and
(92) provide an indicator of COPD exacerbation based on the relationship between the estimated sleep stage and the PAP system signals.

15. A computer program comprising computer program code means which is adapted, when said program is run on a computer, to implement the method of claim 14.
